# EUROPÄISCHE PATENTANMELDUNG

(11) **EP 4 378 919 A1**
(43) Veröffentlichungstag der Anmeldung: **05.06.2024**
(21) Anmeldenummer: 22020582.7
(22) Anmeldetag: 29.11.2022
(51) Int. Cl.: C07C 5/333, C07C 7/09, C07C 7/04, C07C 11/06, C07C 9/08, F25J 3/06

(54) **HERSTELLUNG EINES OLEFINS DURCH DEHYDRIERUNG EINES PARAFFINS**

(71) Anmelder: Linde GmbH, 82049 Pullach (DE)
(72) Erfinder: Berg, Markus, 82049 Pullach (DE); Lewis, Rory, 82049 Pullach (DE)
(74) Vertreter: Fischer, Werner

(57) **Zusammenfassung**

Es wird ein Verfahren zur Herstellung eines Olefins unter Einsatz einer katalytischen Dehydrierung, mit einem Produktionsbetriebsmodus, der umfasst, dass ein Einsatzgemisch, das ein zu dem Olefin kettenlängengleiches Paraffin enthält, unter Erhalt eines Produktgemischs, das einen nicht umgesetzten Teil des Paraffins, das Olefin, und höher als das Olefin siedende Komponenten enthält, der katalytischen Dehydrierung unterworfen wird, das Produktgemisch oder ein Teil hiervon unter Erhalt eines oder mehrerer Kondensate, das oder die zumindest einen Teil des Paraffins, des Olefins, und der höher als das Olefin siedende Komponenten enthält oder enthalten, einer Kondensatabscheidung unterworfen wird, das eine oder die mehreren Kondensate oder ein Teil hiervon unter Verbleib einer Restflüssigkeit, die zumindest einen Teil des Paraffins und des Olefins aus dem einen oder den mehreren Kondensaten enthält, einer Entspannungsverdampfung unterworfen wird, die Restflüssigkeit oder ein Teil hiervon unter Erwärmung einer Wärmeübertragung unterworfen und danach unter Erhalt einer Paraffinfraktion und einer Olefinfraktion einer Paraffinabrennung unterworfen wird, und die Paraffinfraktion oder ein Teil hiervon unter Abkühlung der Wärmeübertragung unterworfen und danach der katalytischen Dehydrierung erneut unterworfen wird. Es ist ein Sonderbetriebsmodus vorgesehen, der umfasst, dass eine das Paraffin enthaltende kryogene Flüssigkeit in einen Verdampfungsbehälter (D4), der bei der Entspannungsverdampfung verwendet wird, entspannt wird, und dass ein bei dieser Entspannung gebildetes Flashgas durch einen bei der erwähnten Wärmeübertragung verwendeten Wärmetauscher (E1) geführt wird. Eine entsprechende Anlage (100) ist ebenfalls Gegenstand der Erfindung.

## Beschreibung

Die Erfindung betrifft ein Verfahren und eine Anlage zur Herstellung eines Olefins durch Dehydrierung eines Paraffins.

### Hintergrund

Verfahren zur Dehydrierung von Paraffinen, insbesondere von Propan zu Propylen, sind bekannt und beispielsweise im Artikel "Propene" in Ullmann's Encyclopedia of Industrial Chemistry, Onlineausgabe, 15. Juni 2000, DOI 10.1002/14356007.a22_211, Abschnitt 4.3., "Propane Dehydrogenation", beschrieben.

Zur Dehydrierung von Propan zu Propylen werden beispielsweise Reaktoren verwendet, die mittels Brennern beheizte Brennkammern aufweisen, durch welche mehrere Reaktionsrohre geführt sind. Die Reaktionsrohre werden von außen durch die Brenner beheizt. Die Reaktionsrohre enthalten einen Katalysator auf einem geeigneten Träger. Geeignete Katalysatoren sind beispielsweise bei Bölt, H., "Dehydrierung von Propan/Butan", Linde Berichte aus Technik und Wissenschaft 66/1991, beschrieben. Durch die Reaktionsrohre wird das zu dehydrierende Propan bzw. ein propanreicher Strom zusammen mit Dampf, sogenanntem Prozessdampf, geführt. Zuvor erfolgt typischerweise eine Vorwärmung mittels Abwärme. Ein dem Reaktor bzw. entsprechenden Reaktionsrohren entnommenes Gasgemisch, nachfolgend auch als Produktgemisch bezeichnet, wird einer Produktaufbereitung zugeführt.

Die Dehydrierung von Propan zu Propylen ist bei der Wahl geeigneter Katalysatoren und Reaktionsbedingungen hochselektiv und kann bei über 90% liegen. Dennoch entstehen immer auch bei der Dehydrierung abgespaltener Wasserstoff sowie geringe Mengen an Nebenprodukten in Form von Methan und anderen Kohlenwasserstoffen. Ferner finden sich in einem entsprechenden Produktgemisch immer auch größere Mengen an nicht zu Propylen umgesetztem Propan und Prozessdampf.

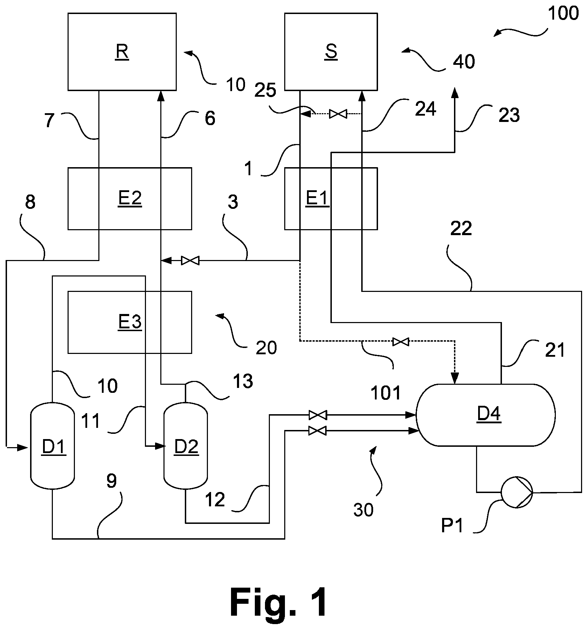

In der EP 3 379 187 A1 sind Beispiele einer entsprechenden Produktaufbereitung in den Figuren 1 und 2 veranschaulicht. Zu weiteren Details wird insbesondere auf die dortigen Absätze [0025] bis [0030] verwiesen. In einer entsprechenden Anordnung wird eine in der EP 3 379 187 A1 als kryogene Behandlungseinrichtung bezeichnete Tieftemperaturtrenneinheit verwendet.

Eine entsprechende Tieftemperaturtrenneinheit ist mit ihren unterschiedlichen Apparaten typischerweise zumindest zum Teil in einer sogenannten Coldbox angeordnet. Unter dem Begriff Coldbox wird dabei üblicherweise eine temperaturisolierende Einhausung verstanden, in der bei tiefen, insbesondere kryogenen Temperaturen betriebene verfahrenstechnische Apparate installiert sind. In einer Coldbox können dabei mehrere Apparate, also beispielsweise mehrere Kolonnen, Abscheider bzw. Separatoren und Wärmetauscher zusammen mit der zugehörigen Verrohrung an einem tragenden Stahlrahmen befestigt werden, welcher außen mit Blechplatten verkleidet wird. Das Innere der auf diese Weise gebildeten Einhausung ist mit Isoliermaterial wie beispielsweise Perlit gefüllt, um einen Wärmeeintrag aus der Umgebung zu verhindern.

Aufgabe der vorliegenden Erfindung ist es unter anderem, Lösungen anzugeben, die es erlauben, eine entsprechende Tieftemperaturtrenneinheit bzw. Coldbox so schnell und gleichmäßig wie möglich in Betrieb zu nehmen, ohne dabei die zulässigen Temperaturgradienten in Bezug auf Zeit und Ort zu überschreiten. Hierbei sollen insbesondere auch Aufwärmszenarien und (ggf. aufgrund extremer klimatischer Bedingungen) das Anfahren im Winter berücksichtigt werden.

### Offenbarung der Erfindung

Vor diesem Hintergrund schlägt die vorliegende Erfindung ein Verfahren und eine Anlage zur Herstellung eines Olefins durch Dehydrierung eines Paraffins mit den Merkmalen der unabhängigen Patentansprüche vor. Ausgestaltungen sind jeweils Gegenstand der abhängigen Patentansprüche und der nachfolgenden Beschreibung.

Die vorliegende Erfindung ermöglicht insbesondere ein schonenderes Anfahren einer entsprechenden Anlage zur Herstellung von Olefinen mit einer Produktaufbereitung. In dem Verfahren kann insbesondere vor dem Beginn des eigentlichen Produktionsprozesses jener Wärmetauscher, der für die Erwärmung einer Kohlenwasserstofffraktion verwendet wird, die nicht umgesetztes Paraffin und das gebildete Olefin enthält, vorteilhaft abgekühlt werden. Ein weiterer Aspekt und Vorteil der vorliegenden Erfindung besteht neben dieser unabhängigen Temperaturabsenkung zur Vorbereitung des Produktionsprozesses in einer Einstellung bzw. Aufrechterhaltung eines akzeptablen Temperaturgradienten in diesem Wärmetauscher durch Einstellen eines "Start-Up-Propankreislaufs" wie unten genauer erläutert im Rahmen eines zweistufigen Verfahrens bzw. Modells.

Die vorliegende Erfindung entfaltet ihre besonderen Vorteile insbesondere im Zusammenhang mit der Dehydrierung von Propan zu Propylen und wird nachfolgend überwiegend unter Bezugnahme hierauf beschrieben. Sie kann grundsätzlich aber auch im Zusammenhang mit anderen Dehydrierungsverfahren, beispielsweise zur Dehydrierung von Butan, verwendet werden.

Betrachtet man beispielsweise die Figur 1 der EP 3 379 187 A1 (die nachfolgenden Bezugszeichen betreffen zunächst diese Figur), so wird dort eine Entspannungsverdampfung von Kondensaten aus den Behältern D1 und D2 in einen Entspannungsbehälter D4 vorgenommen.

Das der Reaktionseinheit R in Form des Stoffstroms 6 zugeführte Paraffin kann in einem Produktionsbetrieb als Stoffstrom 1 aus einer Paraffinabtrennung entnommen werden, der wiederum ein das Paraffin und das entsprechende Olefin enthaltender Stoffstrom 22 bzw. 24 aus dem Behälter D4, der mittels der Pumpe P aus dem Behälter D4 abgezogen wird, zugeführt wird. Dies ist allerdings erst dann möglich, wenn entsprechende Kondensate in den Behältern D1 und D2 vorliegen, d.h. Produktgemisch 7 über einen ausreichend langen Zeitraum gebildet wurde. Ist dies der Fall, entsteht ein entsprechender Paraffinkreislauf.

Solange noch keine Kondensate in den Behältern D1 und D2 gebildet werden, kann auch eine entsprechende Paraffinabtrennung noch nicht mit entsprechendem Einsatz beschickt werden. Daher muss das Paraffin zunächst aus einer anderen Quelle zugeführt werden. Der Wärmetauscher E1 ist in diesem Fall aber noch nicht abgekühlt. Für die Beschickung der Reaktionseinheit R ist dies aber ohne Belang.

Mit beginnender Kondensation sammelt sich zuerst Kondensat in den Behältern D1 und D2, weiterhin muss aber warmes Paraffin zugeführt werden, bis die Paraffinabtrennung ausreichend Paraffin liefert. Damit kommt es zu beträchtlichen Temperaturspannungen im Wärmetauscher E1. Nach Erreichen ausreichender Füllstände in den Behältern D1 und D2 und dem Entspannungsbehälter D4 kann der erwähnte Kreislauf via Pumpe P1 gestartet werden. Damit trifft kalte Flüssigkeit von der Pumpe P1 auf den noch warmen Tauscher E1 und verursacht ebenfalls hohe Temperaturspannungen.

Hier setzt die vorliegende Erfindung an. Sie ermöglicht ein graduelles Temperieren des Wärmetauschers E1 vor dem Beginn des Produktionsprozesses und im Anschluss daran eine Einstellung bzw. Aufrechterhaltung eines akzeptablen Temperaturgradienten in diesem Wärmetauscher E1. Die vorliegende Erfindung ist durch die vorstehende Bezugnahme auf die EP 3 379 187 A1 nicht eingeschränkt.

Vor diesem Hintergrund wird ein Verfahren zur Herstellung eines Olefins unter Einsatz einer katalytischen Dehydrierung vorgeschlagen. Hierbei wird ein Produktionsbetriebsmodus durchgeführt, der umfasst, dass ein Einsatzgemisch, das ein zu dem Olefin kettenlängengleiches Paraffin enthält, unter Erhalt eines Produktgemischs, das einen nicht umgesetzten Teil des Paraffins, das Olefin, und höher als das Olefin siedende Komponenten enthält, der katalytischen Dehydrierung unterworfen wird.

Das Produktgemisch oder ein Teil hiervon wird unter Erhalt eines oder mehrerer Kondensate, das oder die zumindest einen Teil des Paraffins, des Olefins, und der höher als das Olefin siedende Komponenten enthält oder enthalten, einer Kondensatabscheidung unterworfen. Das eine oder die mehreren Kondensate oder ein Teil hiervon wird unter Verbleib einer Restflüssigkeit, die zumindest einen Teil des Paraffins und des Olefins aus dem einen oder den mehreren Kondensaten enthält, einer Entspannungsverdampfung unterworfen. Die Restflüssigkeit oder ein Teil hiervon wird unter Erwärmung einer Wärmeübertragung unterworfen und danach unter Erhalt einer Paraffinfraktion und einer Olefinfraktion einer Paraffinabrennung unterworfen. Die Paraffinfraktion oder ein Teil hiervon wird unter Abkühlung der Wärmeübertragung unterworfen und danach der katalytischen Dehydrierung erneut unterworfen.

Hierbei wird ein Sonderbetriebsmodus vorgeschlagen, der insbesondere nach einer (Wieder-)Inbetriebnahme einer entsprechenden Anlage bzw. vor dem Produktionsbetriebsmodus durchgeführt werden kann, und der umfasst, dass eine das Paraffin enthaltende kryogene Flüssigkeit in einen Verdampfungsbehälter, der bei der Entspannungsverdampfung verwendet wird, entspannt wird, und dass hierbei gebildetes Flashgas durch einen für die Wärmeübertragung verwendeten Wärmetauscher geführt wird. Auf diese Weise wird eine Vorkühlung des für die Wärmeübertragung verwendeten Wärmetauschers (der dem Wärmetauscher E1 gemäß EP 3 379 187 A1 entsprechen kann) möglich. Durch Zuspeisen von Flüssigparaffin und Entspannen in den Verdampfungsbehälter (über den gewählten Druck ist die Temperatur einstellbar) kann das gebildete Flashgas den erforderlichen Temperaturgradienten über den Wärmetauscher einstellen. Der erwähnte Verdampfungsbehälter wird im Produktionsbetriebsmodus als ein Flash- bzw. Ausgasungsbehälter verwendet.

Durch den Einsatz der vorliegenden Erfindung kann die zuvor genannte Aufgabe, die insbesondere darin besteht, die kalten Teile einer entsprechenden Anlage möglichst schnell und gleichmäßig in Betrieb zu nehmen, ohne die Temperaturgradienten in Bezug auf Zeit und Ort zu überschreiten, gelöst werden. Darüber hinaus lassen sich die erwähnten Aufwärmszenarien (ggf. aufgrund extremer klimatischer Bedingungen) und die Inbetriebnahme im Winter berücksichtigen.

Ohne die vorgeschlagenen Maßnahmen sind die geforderten Temperaturgradienten beim Anfahren/Abfahren viel schwieriger zu erreichen, und die Abkühlungsgeschwindigkeit ist erheblich langsamer. Die Erfindung ermöglicht damit auch eine Inbetriebnahme unter extremen Winterbedingungen und eine entsprechende Anlage während sehr heißer Hitzeperioden zu betreiben. In entsprechenden Fällen kann herkömmlicherweise ggf. die Auslegungstemperatur des Zulaufs überschritten werden, was zu einer Abschaltung führen kann.

Insgesamt kann das Anfahren einer entsprechenden Anlage durch den Einsatz der erfindungsgemäß vorgeschlagenen Maßnahmen, die insbesondere unter Verwendung eines zusätzlichen Regelventils zur Entspannung in den Entspannungsbehälter realisiert werden können, beschleunigt und vereinfacht werden. Die Abkühlung der Coldbox kann so erfolgen, dass Entspannungsbehälter und Flüssigproduktpumpen abgekoppelt von anderen Komponenten abgekühlt werden können. Dies führt zu einer sanfteren und kontrollierteren Abkühlung. Durch eine mögliche Druckregelung in dem Entspannungsbehälter kann der Start der Paraffineinspritzung in den kombinierten Feed bei einem niedrigeren Druck erfolgen.

Im Rahmen der vorliegenden Erfindung kann das Temperaturprofil in dem erwähnten Wärmetauscher so eingestellt werden, dass unnötige thermische Belastungen vermieden werden. Dies kann es auch ermöglichen, die Flüssigproduktpumpe im Bypass zu betreiben (unter Verwendung der Leistungsaufnahme als Verdampfer), um die für die Einstellung der Zusammensetzung des Feedabfluss erforderliche Zufuhr von gasförmigem Propan zu ergänzen.

Die in Ausgestaltungen der Erfindung vorgeschlagene Flüssigkeitseinspritzung, d.h. die Entspannung des flüssigen Paraffinstroms in den Entspannungsbehälter, ermöglicht eine frühzeitige Flüssigkeitsansammlung in den vorgeschalteten Behältern und vereinfacht und beschleunigt die Abkühlung der Coldbox. Die Flüssigkeitseinspritzung ermöglicht die Zufuhr von warmem Feed, auch wenn die Coldbox unter extremen Winterbedingungen steht. Auf diese Weise kann die Coldbox im Winter unter Druck gesetzt werden, so dass später warmes Einsatzgas eingeleitet werden kann.

Durch eine optimale Flüssigkeitseinspritzung kann die Einspeisetemperatur in die Coldbox um etwa 2 bis 5 °C gesenkt werden. Dies kann bei heißen Sommerbedingungen in warmen Klimazonen erforderlich sein (Gefahr der Überschreitung der Auslegungstemperatur der Coldbox).

Nochmals zusammengefasst kann durch ein sanfteres und gleichmäßigeres Anfahren die thermische Belastung beim Anfahren und im Normalbetrieb minimiert werden, was zu einer längeren Lebensdauer der betroffenen (Platten-)Wärmetauscher führt. Das Anfahren und der Normalbetrieb können unter extremen klimatischen Bedingungen (Sommer und Winter) erfolgen, was zu einer geringeren thermischen Belastung während des Anfahrens und des Normalbetriebs und somit zu einer längeren Lebensdauer der Wärmetauscher führt.

Ausgestaltungen der vorliegenden Erfindung können umfassen, dass die das Paraffin enthaltende kryogene Flüssigkeit unter Verwendung der Paraffinfraktion oder eines Teils hiervon und/oder unter Verwendung von extern zugeführtem Paraffin gebildet wird. Insbesondere kann es sich dabei um "Make Up"-Paraffin handeln, das auch im Produktionsbetrieb zum Ausgleich des jeweils in der katalytischen Dehydrierung umgesetzten Paraffinanteils verwendet wird.

In einer Ausgestaltung der Erfindung wird ein einstellbarer Anteil der Restflüssigkeit nach der Erwärmung unter Umgehung der Paraffinabtrennung der Paraffinfraktion vor deren Abkühlung zugespeist und zusammen mit dieser der Abkühlung unterworfen und danach der katalytischen Dehydrierung erneut unterworfen. Auf diese Weise kann ein entsprechender "Start-Up-Propankreislauf' geschaffen werden der eine Einstellung bzw. Aufrechterhaltung des Temperaturprofils in dem zur Erwärmung bzw. Abkühlung verwendeten Wärmetauscher ermöglicht.

Im Rahmen der vorliegenden Erfindung kann die Kondensatabscheidung einen ersten Abkühlschritt von einem ersten Temperaturniveau auf ein zweites Temperaturniveau unterhalb des ersten Temperaturniveaus und eine anschließende Abscheidung eines ersten Kondensats sowie einen zweiten Abkühlschritt von dem zweiten Temperaturniveau auf ein drittes Temperaturniveau unterhalb des zweiten Temperaturniveaus und eine anschließende Abscheidung eines zweiten Kondensats umfassen. Die vorliegende Erfindung kann in diesem Zusammenhang insbesondere mit bewährten Produktaufbereitungstechnologien verwendet werden, wie sie bereits unter Bezugnahme auf den Stand der Technik erläutert wurden.

Genauer kann eine bei der Abscheidung des ersten Kondensats verbliebende Gasfraktion oder ein Teil hiervon der Abscheidung des zweiten Kondensats unterworfen werden und eine bei der Abscheidung des zweiten Kondensats verbleibende Gasfraktion oder ein Teil hiervon kann als Kältemittel für den ersten Abkühlschritt und/oder den zweiten Abkühlschritt verwendet werden.

Die Entspannungsverdampfung kann insbesondere auf dem zweiten Temperaturniveau durchgeführt werden, die Restflüssigkeit oder der Teil hiervon, der der Paraffinabtrennung unterworfen wird, kann unter Verwendung der Wärmeübertragung von dem zweiten Temperaturniveau auf das erste Temperaturniveau erwärmt werden, und die Paraffinfraktion oder der Teil hiervon, der der katalytischen Dehydrierung erneut unterworfen wird, kann unter Verwendung der Wärmeübertragung von dem ersten Temperaturniveau auf das zweite Temperaturniveau abgekühlt werden.

Ausgestaltungen der vorliegenden Erfindung umfassen insbesondere, dass die Paraffinfraktion oder der Teil hiervon, der in die Dehydrierung zurückgeführt wird, als ein Kältemittel für den ersten Abkühlschritt verwendet wird.

In Ausgestaltungen der vorliegenden Erfindung kann das erste Temperaturniveau bei 0 bis 50 °C, insbesondere bei 25 bis 35 °C, das zweite Temperaturniveau bei -50 bis 0 °C, insbesondere bei -40 bis -45 °C, beispielsweise bei ca. 42 °C, und das dritte Temperaturniveau bei -150 bis -100 °C, insbesondere bei -110 bis -130 °C, beispielsweise bei 120 °C, liegen.

Wie erwähnt kann das Paraffin Propan sein und das Olefin Propylen sein, und die höher als das Olefin siedenden Komponenten können insbesondere Wasserstoff und Methan umfassen.

Zu dem frühen Zeitpunkt im Anlagenbetrieb, in dem die vorliegende Erfindung gemäß unterschiedlicher Ausgestaltungen zum Einsatz kommt, sind typischerweise keine höher siedende Komponenten in der Anlage vorhanden, bis auf einen Rest an Stickstoff aus einer Inertisierung. Dieser Stickstoff wird aber sogleich durch Verdünnung auf unwesentliche Restkonzentration reduziert. Es ist also typischerweise sogenanntes Rohpropan vorhanden, das bspw. einen Propangehalt von mehr als 95 oder 99 Molprozent aufweist. Die Entspannung, die die Temperatur des Flashgases definiert, wird entsprechend druckabhängig eingestellt, wie bereits oben erwähnt. Die Abkühlung dieses Bereiches ist mit der Ausbildung einer Kondensatphase abgeschlossen (Zweiphasengleichgewicht). Vor allen mit Propan lässt sich eine erforderliche Temperatur gut einstellen.

In Ausgestaltungen der vorliegenden Erfindung kann der Sonderbetriebsmodus auf Grundlage einer Temperaturmessung an oder in zumindest einem in dem Verfahren verwendeten Apparat durchgeführt wird, wobei der Sonderbetriebsmodus beendet wird, wenn ein bei der Temperaturmessung ermittelter Temperaturwert einen vorgegebenen Schwellwert unterschreitet, bzw. beendet werden kann, wenn aus dem Produktionsbetriebsmodus eine ausreichende Menge an Kondensaten aus den verwendeten Abscheidern zur Verfügung steht.

Eine Anlage zur Herstellung eines Olefins, die eine Reaktoreinheit, eine Kondensatabscheidungseinheit, eine Entspannungsverdampfungseinheit und eine Paraffinabtrennungseinheit aufweist, wird ebenfalls vorgeschlagen. Die Anlage ist dafür eingerichtet ist, einen Produktionsbetriebsmodus durchzuführen und in diesem unter Verwendung der Reaktoreinheit ein Einsatzgemisch, das ein zu dem Olefin kettenlängengleiches Paraffin enthält, unter Erhalt eines Produktgemischs, das einen nicht umgesetzten Teil des Paraffins, das Olefin, und höher als das Olefin siedende Komponenten enthält, einer katalytischen Dehydrierung zu unterwerfen, unter Verwendung der Kondensatabscheidungseinheit das Produktgemisch oder einen Teil hiervon unter Erhalt eines oder mehrerer Kondensate, das oder die zumindest einen Teil des Paraffins, des Olefins, und der höher als das Olefin siedende Komponenten enthält oder enthalten, einer Kondensatabscheidung zu unterwerfen, unter Verwendung der Entspannungsverdampfungseinheit das eine oder die mehreren Kondensate oder einen Teil hiervon unter Verbleib einer Restflüssigkeit, die zumindest einen Teil des Paraffins und des Olefins aus dem einen oder den mehreren Kondensaten enthält, einer Entspannungsverdampfung zu unterwerfen, die Restflüssigkeit oder einen Teil hiervon unter Erwärmung einer Wärmeübertragung zu unterwerfen und danach unter Verwendung der Paraffinabtrennungseinheit und unter Erhalt einer Paraffinfraktion und einer Olefinfraktion der Paraffinabrennung zu unterwerfen, und die Paraffinfraktion oder einen Teil hiervon unter Abkühlung der Wärmeübertragung zu unterwerfen und danach der katalytischen Dehydrierung erneut zu unterwerfen.

Eine erfindungsgemäße Anlage ist dafür eingerichtet ist, einen Sonderbetriebsmodus durchzuführen und in diesem eine das Paraffin enthaltende kryogene Flüssigkeit in einen Verdampfungsbehälter in der Entspannungsverdampfungseinheit zu entspannen sowie ein bei dieser Entspannung gebildetes Flashgas durch einen bei der erwähnten Wärmeübertragung verwendeten Wärmetauscher zu führen.

Zu weiteren Merkmalen und Vorteilen einer entsprechenden Anlage und Ausgestaltungen hiervon sei auf die obigen Erläuterungen betreffend das erfindungsgemäß vorgeschlagene Verfahren und seine Ausgestaltungen ausdrücklich verwiesen, da diese hierfür in gleicher Weise gelten.

Entsprechendes gilt auch für eine Anlage, die gemäß einer Ausgestaltung der Erfindung dazu eingerichtet ist, ein Verfahren gemäß einer beliebigen Ausgestaltung der vorliegenden Erfindung durchzuführen.

### Kurze Beschreibung der Zeichnung

Ausführungsformen der Erfindung werden nachfolgend rein beispielhaft unter Bezugnahme auf die beigefügte Zeichnung beschrieben, wobei
Figur 1 eine Anlage gemäß einer Ausgestaltung der Erfindung veranschaulicht.

### Ausführungsformen der Erfindung

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

Die vorliegende Erfindung und Ausgestaltungen hiervon werden nachfolgend unter Bezugnahme auf eine Elektrolyse mit Protonenaustauschmembran erläutert. Wie mehrfach angesprochen, ist die Erfindung jedoch nicht hierauf beschränkt.

In Figur 1 ist eine Anlage gemäß einer Ausgestaltung der vorliegenden Erfindung veranschaulicht und insgesamt mit 100 bezeichnet. Ohne hiermit eine entsprechende Einschränkung zu intendieren, werden nachfolgend die Bezugszeichen aus der EP 3 379 187 A1 verwendet.

In der Anlage 100 Verfahren wird ein Einsatzgemisch 6, das beispielsweise Propan enthält, unter Erhalt eines Produktgemischs 7, das einen nicht umgesetzten Teil des Propans, Propylen, und höher als das Propylen siedende Komponenten, insbesondere Wasserstoff und Methan enthält, einer katalytischen Dehydrierung in einer Reaktionseinheit 10 mit einem Reaktor R unterworfen.

Das Produktgemisch 7 wird in einer insgesamt mit 20 bezeichneten Kondensationseinheit durch einen Wärmetauscher E2 geführt und dann in Form eines Stoffstroms 8 einen Behälter D1 eingespeist. In dem Behälter D1 wird ein erstes Kondensat abgeschieden und in Form eines Stoffstroms 9 einer Entspannungsverdampfung in einen Entspannungsbehälter D4 zugeführt. Die Entspannungsverdampfung ist insgesamt mit 30 angegeben.

Ein in dem Behälter D1 gasförmig verbleibender Anteil wird in Form eines Stoffstroms 10 in einem Wärmetauscher E3 weiter abgekühlt und dann in Form eines Stoffstroms 11 in einen Behälter D2 eingespeist. In dem Behälter D2 wird ein zweites Kondensat abgeschieden und in Form eines Stoffstroms 12 ebenfalls der Entspannungsverdampfung 30 in den Entspannungsbehälter D4 zugeführt.

Ein in dem Behälter D2 gasförmig verbleibender Anteil wird in Form eines Stoffstroms 13 in den Wärmetauschern E3 und E2 erwärmt, dazwischen mit einem Propanstrom 3 vereinigt, und in die Reaktionseinheit 10 zurückgeführt.

Bei der Entspannungsverdampfung verbleibt in dem Entspannungsbehälter D4 eine Restflüssigkeit, die zumindest einen Teil des Paraffins und des Olefins aus dem einen oder den mehreren Kondensaten enthält. Diese wird mittels einer Pumpe P1 in Form eines Stoffstroms 22 durch einen Wärmetauscher E1 geführt und darin unter Erwärmung einer Wärmeübertragung unterworfen. Anschließend erfolgt eine Einspeisung in Form eines Stoffstroms 24 eine insgesamt mit 40 bezeichnete Paraffinabtrennung mit einer Trenneinheit S.

Flashgas aus dem Entspannungsbehälter D4 kann in Form eines Stoffstroms 21 ebenfalls durch den Wärmetauscher E1 geführt und in Form eines Stoffstroms 23 aus dem Verfahren bzw. der Anlage 100 ausgeleitet werden.

In der Paraffinabtrennung 40 werden eine Paraffinfraktion, hier eine Propanfraktion, und eine Olefinfraktion, hier eine Propylenfraktion, gebildet, wobei die Paraffinfraktion in Form eines Stoffstroms 1 in dem Wärmetauscher E1 abgekühlt und dann zur Bildung des Propanstroms 1 verwendet, d.h. der katalytischen Dehydrierung 10 in der Reaktionseinheit R erneut unterworfen wird.

Wie durch einen gestrichelten Strompfeil veranschaulicht, ist in der hier veranschaulichten Ausgestaltung der Erfindung ein Sonderbetriebsmodus vorgesehen, der umfasst, dass eine das Paraffin, hier Propan, enthaltende kryogene Flüssigkeit, wie mit einem Stoffstrom 101 veranschaulicht, in den Verdampfungsbehälter D4, der bei der Entspannungsverdampfung 30 verwendet wird, entspannt wird, wobei das bei dieser Entspannung gebildetes Flashgas wie auch das "reguläre" Flashgas in Form des Stoffstroms 21 durch einen bei der erwähnten Wärmeübertragung verwendeten Wärmetauscher E1 geführt wird.

Zu beliebigen Zeiten, d.h. auch im regulären Betrieb, kann in dem hier veranschaulichten Verfahren bzw. einer entsprechenden Anlage 100 ein einstellbarer Anteil der Restflüssigkeit, d.h. des Stoffstroms 22 nach der Erwärmung in dem Wärmetauscher E1 unter Umgehung der Paraffinabtrennung S zu der Paraffinfraktion bzw. dem Stoffstrom 1 vor deren bzw. dessen Abkühlung in dem Wärmetauscher E1 zugespeist und zusammen mit dieser bzw. diesem der Abkühlung unterworfen und danach der katalytischen Dehydrierung erneut unterworfen werden. Hierdurch kann, mit entsprechenden Vorteilen, der mehrfach erwähnte "Start-Up-Propankreislauf' realisiert und auf diese Weise der Wärmetauscher E1 temperiert werden.

## Patentansprüche

1. Verfahren zur Herstellung eines Olefins unter Einsatz einer katalytischen Dehydrierung, mit einem Produktionsbetriebsmodus, der umfasst, dass
- ein Einsatzgemisch, das ein zu dem Olefin kettenlängengleiches Paraffin enthält, unter Erhalt eines Produktgemischs, das einen nicht umgesetzten Teil des Paraffins, das Olefin, und höher als das Olefin siedende Komponenten enthält, der katalytischen Dehydrierung unterworfen wird,
- das Produktgemisch oder ein Teil hiervon unter Erhalt eines oder mehrerer Kondensate, das oder die zumindest einen Teil des Paraffins, des Olefins, und der höher als das Olefin siedende Komponenten enthält oder enthalten, einer Kondensatabscheidung unterworfen wird,
- das eine oder die mehreren Kondensate oder ein Teil hiervon unter Verbleib einer Restflüssigkeit, die zumindest einen Teil des Paraffins und des Olefins aus dem einen oder den mehreren Kondensaten enthält, einer Entspannungsverdampfung unterworfen wird,
- die Restflüssigkeit oder ein Teil hiervon unter Erwärmung einer Wärmeübertragung unterworfen und danach unter Erhalt einer Paraffinfraktion und einer Olefinfraktion einer Paraffinabrennung unterworfen wird, und
- die Paraffinfraktion oder ein Teil hiervon unter Abkühlung der Wärmeübertragung unterworfen und danach der katalytischen Dehydrierung erneut unterworfen wird,
**gekennzeichnet durch** einen Sonderbetriebsmodus, der umfasst, dass
- eine das Paraffin enthaltende kryogene Flüssigkeit in einen Verdampfungsbehälter (D4), der bei der Entspannungsverdampfung verwendet wird, entspannt wird, wobei ein bei dieser Entspannung gebildetes Flashgas durch einen bei der erwähnten Wärmeübertragung verwendeten Wärmetauscher (E1) geführt wird.

2. Verfahren nach Anspruch 1, bei dem die das Paraffin enthaltende kryogene Flüssigkeit unter Verwendung der Paraffinfraktion oder eines Teils hiervon und/oder unter Verwendung von extern zugeführtem Paraffin gebildet wird.

3. Verfahren nach einem der vorstehenden Ansprüche, bei dem ein einstellbarer Anteil der Restflüssigkeit nach der Erwärmung unter Umgehung der Paraffinabtrennung zu der Paraffinfraktion vor deren Abkühlung zugespeist und zusammen mit dieser der Abkühlung unterworfen und danach der katalytischen Dehydrierung erneut unterworfen wird.

4. Verfahren nach Anspruch einem der vorstehenden Ansprüche, bei dem die Kondensatabscheidung einen ersten Abkühlschritt von einem ersten Temperaturniveau auf ein zweites Temperaturniveau unterhalb des ersten Temperaturniveaus und eine anschließende Abscheidung eines ersten Kondensats sowie einen zweiten Abkühlschritt von dem zweiten Temperaturniveau auf ein drittes Temperaturniveau unterhalb des zweiten Temperaturniveaus und eine anschließende Abscheidung eines zweiten Kondensats umfasst.

5. Verfahren nach Anspruch 4, bei dem eine bei der Abscheidung des ersten Kondensats verbliebende Gasfraktion oder ein Teil hiervon der Abscheidung des zweiten Kondensats unterworfen wird und eine bei der Abscheidung des zweiten Kondensats verbleibende Gasfraktion oder ein Teil hiervon als Kältemittel für den ersten Abkühlschritt und/oder den zweiten Abkühlschritt verwendet wird.

6. Verfahren nach Anspruch 4 oder Anspruch 5, bei dem die Entspannungsverdampfung auf dem zweiten Temperaturniveau durchgeführt wird, bei dem die Restflüssigkeit oder der Teil hiervon, der der Paraffinabtrennung unterworfen wird, unter Verwendung der Wärmeübertragung von dem zweiten Temperaturniveau auf das erste Temperaturniveau erwärmt wird, und bei dem die Paraffinfraktion oder der Teil hiervon, der der katalytischen Dehydrierung erneut unterworfen wird, unter Verwendung der Wärmeübertragung von dem ersten Temperaturniveau auf das zweite Temperaturniveau abgekühlt wird.

7. Verfahren nach einem der Ansprüche 4 bis 6, bei dem die Paraffinfraktion oder der Teil hiervon, der in die Dehydrierung zurückgeführt wird, als ein Kältemittel für den ersten Abkühlschritt verwendet wird.

8. Verfahren nach einem der Ansprüche 4 bis 7, bei dem das erste Temperaturniveau bei 0 bis 50 °C, das zweite Temperaturniveau bei -50 bis 0 °C, und das dritte Temperaturniveau bei -150 bis -100 °C liegt.

9. Verfahren nach einem der Ansprüche 4 bis 8, bei dem das Paraffin Propan und das Olefin Propylen ist, und bei dem die höher als das Olefin siedenden Komponenten Wasserstoff und Methan umfassen.

10. Verfahren nach einem der vorstehenden Ansprüche, bei dem der Sonderbetriebsmodus auf Grundlage einer Temperaturmessung an oder in zumindest einem in dem Verfahren verwendeten Apparat durchgeführt wird, wobei der Sonderbetriebsmodus beendet wird, wenn ein bei der Temperaturmessung ermittelter Temperaturwert einen vorgegebenen Schwellwert unterschreitet.

11. Anlage (100) zur Herstellung eines Olefins, die eine Reaktoreinheit (10), eine Kondensatabscheidungseinheit (20), eine Entspannungsverdampfungseinheit (30) und eine Paraffinabtrennungseinheit (40) aufweist, wobei die Anlage (100) dafür eingerichtet ist, einen Produktionsbetriebsmodus durchzuführen und in diesem
- unter Verwendung der Reaktoreinheit (10) ein Einsatzgemisch, das ein zu dem Olefin kettenlängengleiches Paraffin enthält, unter Erhalt eines Produktgemischs, das einen nicht umgesetzten Teil des Paraffins, das Olefin, und höher als das Olefin siedende Komponenten enthält, einer katalytischen Dehydrierung zu unterwerfen,
- unter Verwendung der Kondensatabscheidungseinheit (20) das Produktgemisch oder einen Teil hiervon unter Erhalt eines oder mehrerer Kondensate, das oder die zumindest einen Teil des Paraffins, des Olefins, und der höher als das Olefin siedende Komponenten enthält oder enthalten, einer Kondensatabscheidung unterworfen wird,
- unter Verwendung der Entspannungsverdampfungseinheit (30), das eine oder die mehreren Kondensate oder einen Teil hiervon unter Verbleib einer Restflüssigkeit, die zumindest einen Teil des Paraffins und des Olefins aus dem einen oder den mehreren Kondensaten enthält, einer Entspannungsverdampfung zu unterwerfen,
- die Restflüssigkeit oder einen Teil hiervon unter Erwärmung einer Wärmeübertragung zu unterwerfen und danach unter Verwendung der Paraffinabtrennungseinheit (40) und unter Erhalt einer Paraffinfraktion und einer Olefinfraktion der Paraffinabrennung zu unterwerfen, und
- die Paraffinfraktion oder einen Teil hiervon unter Abkühlung der Wärmeübertragung zu unterwerfen und danach der katalytischen Dehydrierung erneut zu unterwerfen,
**dadurch gekennzeichnet, dass** die Anlage (100) dafür eingerichtet ist, einen Sonderbetriebsmodus durchzuführen und in diesem
- eine das Paraffin enthaltende kryogene Flüssigkeit in einen Verdampfungsbehälter (D4) in der Entspannungsverdampfungseinheit (30) zu entspannen sowie ein bei dieser Entspannung gebildetes Flashgas durch einen bei der erwähnten Wärmeübertragung verwendeten Wärmetauscher (E1) zu führen.

12. Anlage (100) nach Anspruch 11, die zur Durchführung eines Verfahrens nach einem der Ansprüche 1 bis 10 eingerichtet ist.
